# EUROPEAN PATENT APPLICATION

(11) **EP 3 005 974 A1**
(43) Date of publication of application: **13.04.2016**
(21) Application number: 14803572.8
(22) Date of filing: 28.05.2014
(51) Int. Cl.: A61C 8/00, A61C 13/34, G01N 23/00, G01N 24/00

(54) **DENTAL IMPLANT**

(30) Priority: 31.05.2013 ES 201330796
(71) Applicant: Soler Cegarra, Josep, 08810 Sant Pere de Ribes (ES)
(72) Inventor: Soler Cegarra, Josep, 08810 Sant Pere de Ribes (ES)
(74) Representative: Gallego Jiménez, José Fernando
(86) International application number: PCT/ES2014/070435
(87) International publication number: WO 2014/191602

(57) **Abstract**

This implant (1) includes a cavity (2), which may be open or closed, that has a certain predetermined geometric shape and that is made of the same material as the implant (1), which cavity (2) contains a material (3) whose density is different from that of the material of which the implant (1) is made, and which material (3) possesses certain characteristics suitable for allowing the said cavity (2) and/or the said material (3) to be detected and displayed by means of magnetic resonance, ionizing radiation of the CAT (computed tomography) type, CBCT (cone beam computed tomography), or any other similar radiological examination, with the resulting determination of the position and orientation of the implants in the mouth of the patient.

## Description

### Subject of the invention

A dental implant that includes means suitable for being attached to a bone of a patient, preferably by being screwed into the bone, and for anchoring a dental part or prosthesis to the said bone.

This dental implant possesses certain characteristics that are suitable for allowing its detection by means of an examination employing ionizing radiation or magnetic resonance, so as to serve as a basis for determining the position of the implants and for the subsequent modeling of a dental prosthesis structure based on a computer model, with the generation of a file for the milling of the said structure.

### Scope of applicability of the invention

This invention is applicable in the field of dental implantology.

### Background of the invention

Patent application WO2012113947, held by the present applicant, describes a method for designing dental prosthesis structures on implants, which method includes the modeling of the implants in accordance with a series of stages such as the placement of posts, detectable by x-rays, on the implants installed in the mouth of the patient; the performance of a CAT scan of the mouth of the patient with the posts placed on the implants; the conversion of the outcome of the CAT scan to a three-dimensional computer model for CAD/CAM manipulation; the definition of the posts in the computer model; the CAD/CAM modeling of the structure based on the computer model with the defined posts; and the generation of a file containing positions and orientations for the calculation of the program for milling the prosthesis structures.
This method constitutes a major advance in the design and milling of dental prosthesis structures in comparison to the previously known techniques, which are based on the creation of a plaster mold from an impression taken in the mouth using a tray filled with silicone, because the said method makes it possible to reduce the cumulative errors that occur with the said processes of the prior art.

Nevertheless, in order to simplify even further the work must be done prior to the milling of the prosthesis structure, and to prevent potential dimensional errors resulting from improper placement of the posts on the implants (due, for example, to insufficient tightening), the said assignee of the above-mentioned prior patent WO2012113947 has devised a dental implant that allows the use of the previous method and the CAD/CAM modeling of the structure based directly on the position of the implants in the computer model.

### Description of the invention

The dental implant that is the subject of this invention, which includes means suitable for being attached to a bone of a patient, preferably by being screwed into the bone, and for anchoring a dental part or prosthesis to the said bone, has the particular feature that it includes a cavity, which may be open or closed, that has a certain predetermined geometric shape, that is made of the same material as the implant, and that contains a material whose density is different from that of the material of which the implant is made, and which material possesses certain characteristics suitable for allowing the said cavity and/or the said material to be detected and displayed by means of magnetic resonance, ionizing radiation of the CAT (computed tomography) type, CBCT (cone beam computed tomography), or any other similar radiological examination.

The geometric shape of the cavity must be a predetermined shape, in order to allow its identification and localization by means of a radiological examination, and for the determination of the position and orientation of the corresponding implant in the mouth of the patient.

Notably, the material contained in the cavity may be a fluid or a solid, provided that it possesses the appropriate characteristics for this application.

If the said cavity contains a gaseous material, such as air, the cavity will be detected and displayed by means of any of the above-mentioned examinations, whereas if the said cavity contains a metallic material or any other solid material whose density is different from that of the material of which the implant is made, the said material will be the one that is detected and displayed in the said examinations.

The insertion into the implant of the said material having a different density enables the modeling of the implants based on the actual position of the implants placed in the mouth of the patient, with no need to place on the said implants the above-mentioned posts or other elements that are detectable by x-rays and that may lead to errors during the modeling of the structure of the prosthesis.

This dental implant allows the use of the method for the design of dental prosthesis structures that was described in document WO 2012113947 held by the present applicant, by working directly on the implants in accordance with essentially the same operating procedure but without the intervention or the installation of radiologically visible posts on the implants.

With this dental implant, the method for the design of dental structures, as described in document WO 2012113947 held by the present applicant, consists of the following steps: an examination using magnetic resonance, ionizing radiation of the CAT or CBCT type, or any other similar radiological examination of the mouth of the patient with the implants in position; the acquisition, based on the results of the examination in which the actual positions of the implants were observed, of a three-dimensional computer model, followed by the definition of the implants in the said computer model; and the subsequent CAD/CAM modeling of the prosthetic structure based on the computer model with the previously defined implants, and the generation of a file of positions and orientations for the calculation of the program for milling the prosthesis structure.

### Description of the figures

To complement the present description and in order to facilitate an understanding of the characteristics of the invention, this descriptive specification is accompanied by a set of drawings on which, for illustrative and non-limitative purposes, the following aspects are represented:

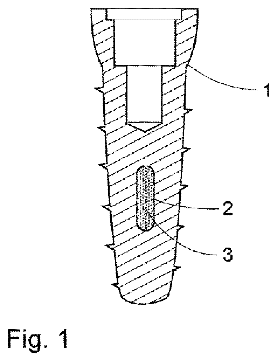

- Figure 1 is an elevation view of one embodiment of the dental implant according to the invention, in section along a vertical midplane.

### Preferred embodiment of the invention

In the embodiment shown in the attached figure, the dental implant referenced in its entirety as (1) includes internally a cavity (2) made of the same material as the implant (1), which cavity (2) contains a material whose density is different from that of the material of which the implant (11) is made.

This material (3) may consist of a fluid, a metal, or a plastic, or may be of any other type that allows the detection and display of the said material (3) or of the said cavity (2) by means of magnetic resonance, ionizing radiation of the CAT or CBCT type, or other similar methods.

With the characteristics described hereinabove, after one or more implants (1) have been installed the mouth of the patient, they will be visible in the results obtained by means of one of the above-mentioned examinations, so as to allow the use of the methods for the design of dental prosthesis structures described in the foregoing background information and belonging to the present applicant, with the examination being performed directly on the implants and with no need to install posts or other radiologically visible elements on the implants.

Inasmuch as a sufficient description of the nature of the invention has been provided, along with one example of a preferred embodiment, it should be noted, for the appropriate purposes, that the materials, shape, size, and arrangement of the elements that have been described may be modified, provided that any such modification does not entail an alteration of the essential characteristics of the invention, as claimed hereinbelow.

## Claims

1. Dental implant, which implant (1) includes means suitable for being attached to a bone of a patient, preferably by being screwed into the bone, and for anchoring a dental part or prosthesis to the said bone, **characterized in that** it includes a cavity (2), which may be open or closed, that has a certain predetermined geometric shape, that is made of the same material as the implant, and that contains a material (3) whose density is different from that of the material of which the implant (1) is made, and which material (3) possesses certain characteristics suitable for allowing the said cavity (2) and/or the said material (3) to be detected and displayed by means of magnetic resonance, ionizing radiation of the CAT (computed tomography) type, CBCT (cone beam computed tomography), or any other similar radiological examination, with the resulting determination of the position and orientation of the implants in the mouth of the patient.

2. Dental implant according to Claim 1, **characterized in that** the material (3) contained in the cavity (2) consists of a fluid or a solid.
